# EUROPÄISCHE PATENTSCHRIFT

(11) **EP 3 356 376 B1**
(45) Veröffentlichungstag und Bekanntmachung des Hinweises auf die Patenterteilung: **07.08.2019**
(21) Anmeldenummer: 16777612.9
(22) Anmeldetag: 26.09.2016
(51) Int. Cl.: C07F 9/40

(54) **VERFAHREN ZUR HERSTELLUNG VON PHOSPHINATEN**
METHOD FOR THE PREPARATION OF PHOSPHINATES
PROCEDE DE FABRICATION DE PHOSPHINATE

(30) Priorität: 29.09.2015 EP 15187469
(43) Veröffentlichungstag der Anmeldung: 08.08.2018
(73) Patentinhaber: BASF SE, 67056 Ludwigshafen am Rhein (DE)
(72) Erfinder: RESSEL, Hans-Joachim, 65795 Hattersheim (DE)
(74) Vertreter: BASF IP Association
(86) Internationale Anmeldenummer: PCT/EP2016/072786
(87) Internationale Veröffentlichungsnummer: WO 2017/055193

(56) Entgegenhaltungen:
- US-A- 4 168 963
- US-A- 4 599 207
- JABLONKAI ERZSÉBET ET AL: "T3P TM -assisted esterification and amidation of phosphinic acids", TETRAHEDRON, ELSEVIER SCIENCE PUBLISHERS, AMSTERDAM, NL, Bd. 70, Nr. 44, 17. September 2014 (2014-09-17), Seiten 8280-8285, XP029051490, ISSN: 0040-4020, DOI: 10.1016/J.TET.2014.09.021

## Beschreibung

Die vorliegende Erfindung betrifft primär ein Verfahren zur Herstellung von Phosphinaten (Phosphonigsäure-mono-estern) der nachfolgend definierten Formel (I) und deren Verwendung zur Herstellung von biologisch aktiven Substanzen, die im pharmazeutischen bzw. agrochemischen Bereich eingesetzt werden können, bevorzugt zur Herstellung von phosphorhaltigen Aminosäuren.

Phosphinate (Phosphonigsäure-mono-ester), wie beispielsweise Alkylphosphinate (Alkylphosphonigsäure-mono-ester) sind wertvolle Zwischenprodukte auf verschiedenen technischen Gebieten, insbesondere zur Herstellung biologisch aktiver Substanzen, die im pharmazeutischen bzw. agrochemischen Bereich eingesetzt werden können.

Zum Beispiel beschreibt US 4,168,963 diverse phosphorhaltige herbizid wirksame Verbindungen, von denen insbesondere Phosphinothricin (2-Amino-4-[hydroxy(methyl)phosphinoyl]-butansäure; Kurzbezeichnung ("common name"): Glufosinate, nachfolgend Glufosinat) bzw. dessen Salze kommerzielle Bedeutung im Bereich der Agrochemie (Agrarchemie) erlangt haben.

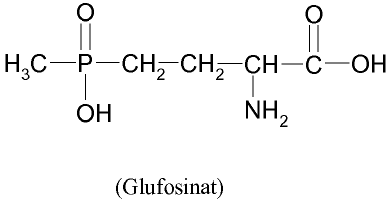

Methoden zur Herstellung von Zwischenprodukten zur Synthese solcher phosphorhaltiger herbizid wirksamer Verbindungen, insbesondere von Glufosinat, sind beispielsweise in US 4,521,348, US 4,599,207 und US 6,359,162B1 beschrieben.

Die Herstellung Monoalkylphosphinaten ist dem Fachmann bekannt und kann nach literaturbekannten Verfahren erfolgen, z.B. gemäß US 3,914,345, US 4,474,711 oder US 5,128,495.

Die Herstellung von Monoalkylphosphinaten ausgehend von bestimmten Phosphorhalogenverbindungen ist beispielsweise in US 4,485,052, GB 1461 376 bzw. GB 1 490 835 beschrieben.

Die Herstellung von Dialkyl-alkylphosphoniten ist beispielsweise beschrieben in US 5,166,385.

Ferner sind auch Verseifungsreaktionen von bestimmten Dialkyl alkylphosphoniten zu Alkylphosphinsäuren oder deren Monoestern bekannt, wie z.B. beschrieben in Applied Spectroscopy 1968, 22, 95-98, J. Med. Chem. 1988, 31, 204-212, J. of General Chem. of the USSR 1962, 32, 3288-3296, Synthetic Communications 2003, 33, 1665-1674 und J. Organomet. Chem. 1997, 529, 135-142. J. Am. Chem. Soc. 1958, 80, 5937-5940 beschreibt die Umesterungsreaktion von bestimmten Dialkyl alkylphosphoniten.

Tetrahedron 2014, 70(44), 8280-8285 beschreibt die Herstellung von Phosphinaten durch Veresterung von Phosphinsäurederivaten.

Die Verfahren aus dem Stand der Technik zur Herstellung von Alkylphosphinaten (Alkylphosphonigsäure-mono-estern) weisen jedoch noch Nachteile auf, wie beispielsweise unzureichende Reinheiten und/oder Ausbeuten an Alkylphosphinaten (Alkylphosphonigsäure-monoestern), einen zu hohen Anteil an Koppel- oder Nebenprodukten, einen zu hohen Aufwand bei der Reinigung bzw. Isolierung der Alkylphosphinate (Alkylphosphonigsäure-mono-ester) und/oder zu schwierige verfahrens-bzw. anlagentechnisch Reaktionsbedingungen. Alkylphosphonigsäure-mono-C₁-C₃-ester sind wegen ihrer geringen Stabilität und ihres Gefahrenpotenzials im technischen (industriellen) Maßstab nicht bevorzugt zu verwenden. Bei der großtechnischen Synthese von Glufosinat-Ammonium ist daher das Butyl methylphosphinat ein wichtiges Zwischenprodukt.

Aufgabe der vorliegenden Erfindung war es daher ein Verfahren zur Herstellung von Phosphinaten (Phosphonigsäure-mono-estern) zu finden, insbesondere von Alkylphosphinaten (Alkylphosphonigsäure-mono-estern), welches die Phosphinate (Phosphonigsäure-mono-ester) in verbesserter Ausbeute liefert und/oder einen geringeren Anteil an Koppel- oder Nebenprodukten ergibt, und zudem dabei vorzugsweise eine verbesserte Reaktionsführung ermöglicht, beispielsweise in Bezug auf sicherheits-, umwelt- und/oder qualitätsrelevante Aspekte, und dadurch vorzugsweise auch für die Durchführung im technischen (industriellen) Maßstab geeignet sein.

Das nachfolgend beschriebene erfindungsgemäße Verfahren erfüllt diese Aufgabe.

Gegenstand der vorliegenden Erfindung ist ein Verfahren zur Herstellung von Phosphinaten (Phosphonigsäure-mono-estern) der Formel (I) 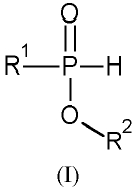 dadurch gekennzeichnet, dass eine Verbindung der Formel (II) 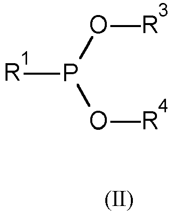 mit einer Verbindung der Formel (III)

R²-OH (III)

wobei jeweils gilt:
R¹ bedeutet (C₁-C₁₂)-Alkyl, (C₁-C₁₂)-Haloalkyl, (C₆-C₁₀)-Aryl, (C₆-C1₀)-Haloaryl, (C₇-C₁₀)-Aralkyl, (C₇-C₁₀)-Haloaralkyl, (C₄-C₁₀)-Cycloalkyl oder (C₄-C₁₀)-Halocycloalkyl,
R² bedeutet (C₃-C₁₂)-Alkyl, (C₃-C₁₂)-Haloalkyl, (C₆-C₁₀)-Aryl, (C₆-C₁₀)-Haloaryl, (C₇-C₁₀)-Aralkyl, (C₇-C₁₀)-Haloaralkyl, (C₄-C₁₀)-Cycloalkyl oder (C₄-C₁₀)-Halocycloalkyl,
R³ und R⁴ bedeuten jeweils unabhängig voneinander Methyl oder Ethyl,
in Gegenwart eines sauren Katalysators und in Gegenwart von Wasser umgesetzt wird.

Durch die Umsetzung der Verbindung der Formel (II) mit einem Alkohol der Formel (III) unter gleichzeitiger Anwesenheit einer Mindestmenge an Wasser und in Gegenwart eines sauren Katalysators werden die gewünschten Phosphinate (Phosphonigsäure-mono-ester) der Formel (I) mit dem erfindungsgemäßen Verfahren in hervorragender Ausbeute und in sehr hoher Reinheit erhalten.

In dem erfindungsgemäßen Verfahren erfolgen in situ partielle Hydrolyse und Umesterung der Verbindungen der Formel (II), vorzugsweise in einer Eintopfreaktion. In dem erfindungsgemäßen Verfahren, insbesondere in einer der als bevorzugt bzw. als besonders bevorzugt bezeichneten Ausgestaltungen des erfindungsgemäßen Verfahrens, werden die Phosphinate (Phosphonigsäure-mono-ester) der Formel (I) in nahezu quantitativer Ausbeute und in sehr hoher Reinheit erhalten. Das erfindungsgemäße Verfahren ist (verfahrenstechnisch) sehr einfach und zur Durchführung im großtechnischen Maßstab geeignet.

Insgesamt entstehen in dem erfindungsgemäßen Verfahren, auch in dem nachfolgend beschriebenen Verfahren zur Herstellung von Glufosinat, weniger unerwünschte Nebenkomponenten, so dass diese Verfahren effizienter und energiesparender sind.

Die jeweiligen Alkylreste der Reste R¹ und R² können im Kohlenstoffgerüst jeweils geradkettig oder verzweigtkettig (verzweigt) sein.

Beispielsweise ist dabei der Ausdruck "(C₁-C₄)-Alkyl" die Kurzschreibweise für einen Alkylrest mit 1 bis 4 Kohlenstoffatomen, d.h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z.B. "(C₁-C₆)-Alkyl" umfassen entsprechend auch gradkettige oder verzweigte Alkylreste mit einer größeren Zahl von C-Atomen, d.h. auch die Alkylreste mit 5 und 6 C-Atomen.

"Halogen" bezieht sich vorzugsweise auf die Gruppe bestehend aus Fluor, Chlor, Brom und Iod. Haloalkyl, Haloaryl, Haloaralkyl und Halocycloalkyl bedeuten durch gleiche oder verschiedene Halogenatome, vorzugsweise aus der Gruppe Fluor, Chlor und Brom, insbesondere aus der Gruppe Fluor und Chlor, teilweise oder vollständig substituiertes Alkyl, Aryl, Aralkyl bzw. Cycloalkyl. So umfasst Haloalkyl z.B. Monohaloalkyl (= Monohalogenalkyl), Dihaloalkyl (= Dihalogenalkyl), Trihaloalkyl (= Trihalogenalkyl), oder auch Perhaloalkyl, wie beispielsweise CF₃, CHF₂, CH₂F, CF₃CF₂, CH₂FCHCl, CCl₃, CHCl₂, CH₂CH₂Cl. Entsprechendes gilt für die anderen durch Halogen substituierte Reste.

Das erfindungsgemäße Verfahren betrifft vorzugsweise die Herstellung von Phosphinaten (Phosphonigsäure-mono-estern) der Formel (I)
wobei jeweils
- R¹: (C₁-C₆)-Alkyl, (C₁-C₆)-Haloalkyl, (C₆-C₈)-Aryl, (C₆-C₈)-Haloaryl, (C₇-C₁₀)-Aralkyl, (C₇-C₁₀)-Haloaralkyl, (C₅-C₈)-Cycloalkyl oder (C₅-C₈)-Halocycloalkyl bedeutet,
und
- R²: (C₃-C₈)-Alkyl, (C₃-C₈)-Haloalkyl, (C₆-C₈)-Aryl, (C₆-C₈)-Haloaryl, (C₇-C₁₀)-Aralkyl, (C₇-C₁₀)-Haloaralkyl, (C₅-C₈)-Cycloalkyl oder (C₅-C₈)-Halocycloalkyl bedeutet.

Das erfindungsgemäße Verfahren betrifft bevorzugt die Herstellung von Phosphinaten (Phosphonigsäure-mono-estern) der Formel (I)
wobei jeweils
- R¹: (C₁-C₄)-Alkyl, (C₁-C₄)-Haloalkyl oder (C₆-C₈)-Aryl, bedeutet, bevorzugt Methyl oder Ethyl,
und
- R²: (C₃-C₆)-Alkyl oder (C₃-C₆)-Haloalkyl bedeutet, bevorzugt (C₃-C₆)-Alkyl, dabei wiederum bevorzugt C₄-Alkyl oder C₅-Alkyl.

Das erfindungsgemäße Verfahren betrifft insbesondere die Herstellung von bestimmten Alkylphosphinaten (Alkylphosphonigsäure-mono-estern) der Formel (I), wobei besonders bevorzugt in Formel (I)
R¹ Methyl bedeutet, und
R² (C₃-C₆)-Alkyl bedeutet, dabei wiederum besonders bevorzugt (C₄-C₅)-Alkyl.

Die nachfolgenden Ausführungen und die als bevorzugt bzw. besonders bevorzugt gekennzeichneten Ausgestaltungen des erfindungsgemäßen Verfahrens gelten insbesondere für die Herstellung einer Verbindung der Formel (I), in der R¹ Methyl bedeutet und R² (C₃-C₆)-Alkyl bedeutet.

Ein vorteilhaftes erfindungsgemäßes Verfahren ist dadurch gekennzeichnet, dass die insgesamt eingesetzte Menge an Wasser mindestens 0,8 molare Äquivalente beträgt, bevorzugt mindestens 0,9 molare Äquivalente, weiter bevorzugt mindestens 0,95 molare Äquivalente, jeweils bezogen auf die insgesamt eingesetzte Menge an Verbindungen der Formel (II).

Ein bevorzugtes erfindungsgemäßes Verfahren ist dadurch gekennzeichnet, dass die insgesamt eingesetzte Menge an Wasser 1 bis 5 molare Äquivalente beträgt, bezogen auf die insgesamt eingesetzte Menge an Verbindungen der Formel (II).

Ein besonders bevorzugtes erfindungsgemäßes Verfahren ist dadurch gekennzeichnet, dass die insgesamt eingesetzte Menge an Wasser 1 bis 3 molare Äquivalente beträgt, ganz besonders bevorzugt 1 bis 2 molare Äquivalente, jeweils bezogen auf die insgesamt eingesetzte Menge an Verbindungen der Formel (II).

Ein bevorzugtes erfindungsgemäßes Verfahren ist dadurch gekennzeichnet, dass die insgesamt eingesetzte Menge an Verbindungen der Formel (III) 1 bis 25 molare Äquivalente beträgt, vorzugsweise 2 bis 20 molare Äquivalente, jeweils bezogen auf die insgesamt eingesetzte Menge an Verbindungen der Formel (II).

Ein besonders bevorzugtes erfindungsgemäßes Verfahren ist dadurch gekennzeichnet, dass die insgesamt eingesetzte Menge an Verbindungen der Formel (III) 3 bis 15 molare Äquivalente beträgt, vorzugsweise 4 bis 12 molare Äquivalente, bevorzugt 5 bis 10 molare Äquivalente, jeweils bezogen auf die insgesamt eingesetzte Menge an Verbindungen der Formel (II).

Ein vorzugsweise zurückgewonnener Überschuss der Verbindung (III) kann anschließend ohne weitere Reinigung wieder in dieselbe Reaktion eingesetzt werden.

In einer bevorzugten Ausgestaltung wird in dem erfindungsgemäßen Verfahren vorzugsweise zunächst eine Teilmenge der insgesamt eingesetzten Gesamtmenge der Alkohole der Formel (III) in die Reaktion eingesetzt. Es wird bevorzugt zunächst ein Anteil von 20 bis 80 Gew.-%, bevorzugt ein Anteil von 30 bis 70 Gew.-%, weiter bevorzugt ein Anteil von 40 bis 60 Gew.-%, der insgesamt eingesetzten Gesamtmenge der Alkohole der Formel (III) mit den Verbindungen der Formel (II), Wasser und saurem Katalysator vermischt und umgesetzt, bevor die restliche Menge der insgesamt eingesetzten Gesamtmenge der Alkohole der Formel (III) in die entstandene Reaktionsmischung zugegeben wird.

Ein bevorzugtes erfindungsgemäßes Verfahren ist dadurch gekennzeichnet, dass der pKs-Wert des sauren Katalysators unter Standardbedingungen (273,15 K und 100 kPa) kleiner als 3 ist, vorzugsweise kleiner als 2 ist, und bevorzugt kleiner als 1 ist.

Bevorzugte saure Katalysatoren in einem erfindungsgemäßen Verfahren sind dabei ausgewählt aus H₃PO₃, H₂SO₄, HC1, HBr, HClO₄, Dichloressigsäure, Trichloressigsäure, Trifluoressigsäure, Methansulfonsäure, Trifluormethansulfonsäure, Benzolsulfonsäure, und p-Toluolsulfonsäure.

Bevorzugte saure heterogene (Feststoff)Katalysatoren in einem erfindungsgemäßen Verfahren sind ausgewählt aus sauren Ionenaustauschern, sauren Polysiloxanen und sauren Zeolithen

Ein bevorzugtes erfindungsgemäßes Verfahren ist daher dadurch gekennzeichnet, dass einer oder der saure Katalysator ausgewählt ist aus der Gruppe bestehend aus H₃PO₃, H₂SO₄, HC1, HBr, HClO₄, Dichloressigsäure, Trichloressigsäure, Trifluoressigsäure, Methansulfonsäure, Trifluormethansulfonsäure, Benzolsulfonsäure, p-Toluolsulfonsäure, sauren Ionenaustauschern, sauren Polysiloxanen und sauren Zeolithen.

Ein besonders bevorzugtes erfindungsgemäßes Verfahren ist dadurch gekennzeichnet, dass der pKs-Wert des sauren Katalysators unter Standardbedingungen (273,15 K und 100 kPa) kleiner als 0 (null) ist, und vorzugsweise kleiner als -1 ist.

Besonders bevorzugte saure Katalysatoren sind dabei ausgewählt aus H₂SO₄, HCl, Methansulfonsäure, Trifluormethansulfonsäure und p-Toluolsulfonsäure.

Besonders bevorzugte saure heterogene (Feststoff)Katalysatoren sind solche mit Sulfonsäuregruppen, d.h. -SO₃H-Gruppen.

Die in den erfindungsgemäßen Verfahren insgesamt eingesetzte Menge (Gesamtmenge) des oder der sauren Katalysatoren ist nicht entscheidend für die Durchführung des erfindungsgemäßen Verfahrens, sie beträgt vorzugsweise 0,1 bis 10 Gew.-%, bevorzugt 0,5 bis 5 Gew.-%, jeweils bezogen auf die insgesamt eingesetzte Menge (Gesamtmenge) an Verbindungen der Formel (II).

Eine besonderer Vorteil bzw. eine besonders bevorzugte Ausgestaltung des erfindungsgemäßen Verfahrens ist dadurch gekennzeichnet, dass die Umsetzung als Eintopfreaktion durchgeführt wird, d.h. dass eine besonders bevorzugte Umsetzung der Verbindungen (II) und (III) zu Verbindung (I) erfindungsgemäß ohne Isolierung von Zwischenprodukten erfolgt.

Das erfindungsgemäße Verfahren wird vorzugsweise in der Weise durchgeführt, dass die Umsetzung bei einer Temperatur im Bereich von 30 bis 140 °C erfolgt, bevorzugt bei einer Temperatur im Bereich von 40 bis 130 °C, weiter bevorzugt bei einer Temperatur im Bereich von 50 bis 120 °C, und besonders bevorzugt bei einer Temperatur im Bereich von 60 bis 110 °C.

Das erfindungsgemäße Verfahren ermöglicht die Herstellung der Phosphinate (Phosphonigsäure-mono-ester) der Formel (I) unter milden Reaktionsbedingungen bei einfacher verfahrens- bzw. anlagentechnischer Durchführbarkeit. Dadurch können die Phosphinate (Phosphonigsäure-mono-ester) der Formel (I) prozesstechnisch leichter, in besseren Ausbeuten und in hoher Reinheit erhalten werden.

Die Reinheit der gewünschten Produkte der Formel (I) nach Reinigung, zum Beispiel nach destillativer Reinigung, liegt regelmäßig bei mehr als 95%.

Das erfindungsgemäße Verfahren eignet sich daher insbesondere für die Durchführung im technischen (industriellen) Maßstab, d.h. dass vorzugsweise das erfindungsgemäße Verfahren in der Weise durchgeführt wird, dass mindestens eine Menge von 100 kg an Verbindungen der Formel (II) in das erfindungsgemäße Verfahren eingesetzt wird, bevorzugt mindestens eine Menge von 250 kg an Verbindungen der Formel (II), weiter bevorzugt mindestens eine Menge von 500 kg an Verbindungen der Formel (II).

Die gebildeten Phosphinate (Phosphonigsäure-mono-ester) der Formel (I) können als Ausgangsstoffe zur Synthese von phosphorhaltigen Aminosäuren wie beispielsweise Glufosinat und Glufosinat-Salzen verwendet werden.

Bevorzugte Ausgestaltungen des erfindungsgemäßen Verfahrens zur Herstellung der Verbindungen der Formel (I) durch Umsetzung einer Verbindung der Formel (II) mit dem Alkohol der Formel (III), sind dadurch gekennzeichnet,
dass die insgesamt eingesetzte Menge an Wasser 1 bis 3 molare Äquivalente beträgt, bezogen auf die insgesamt eingesetzte Menge an Verbindungen der Formel (II),
und
die insgesamt eingesetzte Menge an Verbindungen der Formel (III) 2 bis 20 molare Äquivalente beträgt, weiter bevorzugt beträgt die insgesamt eingesetzte Menge an Verbindungen der Formel (III) 3 bis 15 molare Äquivalente, jeweils bezogen auf die insgesamt eingesetzte Menge an Verbindungen der Formel (II).

Weiter bevorzugte Ausgestaltungen des erfindungsgemäßen Verfahrens zur Herstellung der Verbindungen der Formel (I) durch Umsetzung einer Verbindung der Formel (II) mit dem Alkohol der Formel (III), sind dadurch gekennzeichnet,
dass die insgesamt eingesetzte Menge an Wasser 1 bis 2 molare Äquivalente beträgt, bezogen auf die insgesamt eingesetzte Menge an Verbindungen der Formel (II),
und
die insgesamt eingesetzte Menge an Verbindungen der Formel (III) 4 bis 12 molare Äquivalente beträgt, weiter bevorzugt beträgt die insgesamt eingesetzte Menge an Verbindungen der Formel (III) 5 bis 10 molare Äquivalente, jeweils bezogen auf die insgesamt eingesetzte Menge an Verbindungen der Formel (II).

Besonders bevorzugte Ausgestaltungen des erfindungsgemäßen Verfahrens zur Herstellung der Verbindungen der Formel (I) durch Umsetzung einer Verbindung der Formel (II) mit dem Alkohol der Formel (III), sind dadurch gekennzeichnet,
dass die insgesamt eingesetzte Menge an Wasser 1 bis 3 molare Äquivalente beträgt, bezogen auf die insgesamt eingesetzte Menge an Verbindungen der Formel (II),
die insgesamt eingesetzte Menge an Verbindungen der Formel (III) 2 bis 20 molare Äquivalente beträgt, weiter bevorzugt beträgt die insgesamt eingesetzte Menge an Verbindungen der Formel (III) 3 bis 15 molare Äquivalente, jeweils bezogen auf die insgesamt eingesetzte Menge an Verbindungen der Formel (11),
der oder die sauren Katalysatoren ausgewählt sind aus der Gruppe bestehend aus H₂SO₄, HCl, Methansulfonsäure, Trifluormethansulfonsäure, p-Toluolsulfonsäure und sauren heterogene (Feststoff)Katalysatoren mit Sulfonsäuregruppen, d.h. -SO₃H-Gruppen,
die Umsetzung bei einer Temperatur im Bereich von 30 bis 140 °C erfolgt, bevorzugt bei einer Temperatur im Bereich von 40 bis 130 °C,
und vorzugsweise die insgesamt eingesetzte Menge (Gesamtmenge) des oder der sauren Katalysatoren 0,1 bis 10 Gew.-% beträgt, bevorzugt 0,5 bis 5 Gew.-%, jeweils bezogen auf die insgesamt eingesetzte Menge (Gesamtmenge) an Verbindungen der Formel (II).

Insbesondere bevorzugte Ausgestaltungen des erfindungsgemäßen Verfahrens zur Herstellung der Verbindungen der Formel (I) durch Umsetzung einer Verbindung der Formel (II) mit dem Alkohol der Formel (III), sind dadurch gekennzeichnet,
dass die insgesamt eingesetzte Menge an Wasser 1 bis 2 molare Äquivalente beträgt, bezogen auf die insgesamt eingesetzte Menge an Verbindungen der Formel (II),
die insgesamt eingesetzte Menge an Verbindungen der Formel (III) 3 bis 15 molare Äquivalente beträgt, weiter bevorzugt beträgt die insgesamt eingesetzte Menge an Verbindungen der Formel (III) 4 bis 12 molare Äquivalente, jeweils bezogen auf die insgesamt eingesetzte Menge an Verbindungen der Formel (11),
der oder die sauren Katalysatoren ausgewählt sind aus der Gruppe bestehend aus H₂SO₄, HCl, Methansulfonsäure, Trifluormethansulfonsäure, p-Toluolsulfonsäure und sauren heterogene (Feststoff)Katalysatoren mit Sulfonsäuregruppen, d.h. -SO₃H-Gruppen,
die Umsetzung bei einer Temperatur im Bereich von 50 bis 120 °C erfolgt, bevorzugt bei einer Temperatur im Bereich von 60 bis 110 °C,
und
die insgesamt eingesetzte Menge (Gesamtmenge) des oder der sauren Katalysatoren 0,5 bis 5 Gew.-% beträgt, bezogen auf die insgesamt eingesetzte Menge (Gesamtmenge) an Verbindungen der Formel (II).

Das erfindungsgemäße Verfahren kann sowohl in diskontinuierlicher Prozessführung (z.B. in semi-Batch Fahrweise) als auch in kontinuierlicher Prozessführung (z.B. im kontinuierlich betriebenen Rührkessel) durchgeführt werden.

Das erfindungsgemäße Verfahren wird vorzugsweise unter Inertisierung, bevorzugt in einer Schutzgasatmosphäre durchgeführt. Bevorzugte Schutzgase sind dabei Stickstoff bzw. Argon.

Es ist ferner möglich, das erfindungsgemäße Verfahren unter Überdruck oder unter vermindertem Druck durchzuführen.

Das erfindungsgemäße Verfahren kann in einem inerten Verdünnungsmittel oder verdünnungsmittelfrei durchgeführt werden.

Sofern das erfindungsgemäße Verfahren in einem inerten, d.h. einem unter Reaktionsbedingungen stabilen, Verdünnungsmittel durchgeführt wird, wird das Verdünnungsmittel vorzugsweise ausgewählt aus der Gruppe bestehend aus Kohlenwasserstoffen (dabei bevorzugt sind Alkane und aromatische Kohlenwasserstoffe), halogenierten Kohlenwasserstoffen (dabei bevorzugt sind halogenierte Alkane und halogenierte aromatische Kohlenwasserstoffe), Ethern (dabei bevorzugt sind cyclische Ether, Alkylalkylether und Arylalkylether), Carbonsäureestern (dabei bevorzugt sind Alkylalkylester und Arylalkylester) und Carbonsäureamiden, und deren Gemischen.

Als optionale Verdünnungsmittel in dem erfindungsgemäßen Verfahren sind inerte organische Lösungsmittel einsetzbar, wie beispielsweise Heptan, Toluol, Xylol, Ethylbenzol, Chlorbenzol, Dichlorbenzol, Anisol, Dimethylformamid (DMF), Dimethylacetamid, *N*-Methyl-2-pyrrolidon (NMP), oder Mischungen dieser organischen Lösungsmittel.

Bevorzugte Verdünnungsmittel sind dabei Heptan, Toluol, Xylol, Ethylbenzol, Cumol, Chlorbenzol, Dichlorbenzol, Anisol, und deren Mischungen.

Weiter bevorzugt bildet das Verdünnungsmittel mit dem oder den aus den Resten R³ bzw. R⁴ der Verbindung der Formel (II) bei dem erfindungsgemäßen Verfahren entstehenden Alkoholen Methanol und/oder Ethanol ein azeotropes Gemisch, welches vorzugsweise destillativ aus dem Reaktionsmischung entfernt, d.h. abdestilliert werden kann. Diesbezüglich bevorzugte Verdünnungsmittel sind aromatische Kohlenwasserstoffe, dabei wiederum bevorzugt Alkylbenzole wie Toluol, Xylol und/oder Ethylbenzol.

In einer bevorzugten Ausgestaltung wird das erfindungsgemäße Verfahren ohne Zugabe eines Verdünnungsmittels durchgeführt.

Bei Glufosinat-Salzen im Rahmen der vorliegenden Erfindung handelt es sich vorzugsweise um Ammonium-Salze, Phosphonium-Salze, Sulfonium-Salze, Alkali-Salze und Erdalkali-Salze von Glufosinat.

Insbesondere bevorzugt im Rahmen der vorliegenden Erfindung sind Glufosinat, Glufosinat-Natrium bzw. Glufosinat-Ammonium.

Schließlich betrifft die vorliegende Erfindung auch die Verwendung einer gemäß einem erfindungsgemäßen Verfahren hergestellten Verbindung der Formel (I), wie vorstehend definiert, zur Herstellung von biologisch aktiven Substanzen, die im pharmazeutischen bzw. agrochemischen Bereich eingesetzt werden können, bevorzugt zur Herstellung von phosphorhaltigen Aminosäuren, insbesondere zur Herstellung von Glufosinat bzw. von Glufosinat-Salzen, dabei insbesondere von Glufosinat, Glufosinat-Natrium oder Glufosinat-Ammonium.

Das Verfahren zur Herstellung von Glufosinat und/oder Glufosinat-Salzen kann in ähnlicher Weise erfolgen, wie beispielsweise in US 4,521,348 beschrieben.

Ferner betrifft die vorliegende Erfindung ein Verfahren zur Herstellung von biologisch aktiven Substanzen, die im pharmazeutischen bzw. agrochemischen Bereich eingesetzt werden können, bevorzugt zur Herstellung von phosphorhaltigen Aminosäuren, insbesondere zur Herstellung von Glufosinat bzw. von Glufosinat-Salzen, dabei insbesondere von Glufosinat, Glufosinat-Natrium oder Glufosinat-Ammonium, umfassend die folgenden Schritte (a) und (b):
(a) Herstellung einer gemäß einem erfindungsgemäßen Verfahren hergestellten Verbindung der Formel (I), wie vorstehend definiert, bevorzugt wie vorstehend bevorzugt bzw. besonders bevorzugt definiert,
(b) Verwendung der in Schritt (a) hergestellten Verbindung der Formel (I) zur Herstellung von biologisch aktiven Substanzen, die im pharmazeutischen bzw. agrochemischen Bereich eingesetzt werden können, bevorzugt zur Herstellung von phosphorhaltigen Aminosäuren, insbesondere zur Herstellung von Glufosinat bzw. von Glufosinat-Salzen, dabei insbesondere von Glufosinat, Glufosinat-Natrium oder Glufosinat-Ammonium.

Die nachfolgenden Beispiele veranschaulichen die vorliegende Erfindung.

### Beispiele:

Sofern nicht anders angegeben beziehen sich alle Angaben auf das Gewicht.

### Beispiel 1: 1-Butyl methylphosphinat (Methanphosphonigsäure-mono-n-butylester)

38,45 g (0,2684 mol) Diethyl methylphosphonit (Reinheit 95%) und 1,5 g Amberlyst® 15 (stark saurer Katalysator, Kunstharz-Ionenaustauscher) wurden in 200 ml Toluol in einem Rührkolben unter ArgonAtmosphäre vorgelegt. Unter Rühren wurden 4,95 g (0,275 mol) Wasser sowie 50 g (0,675 mol) 1-Butanol zugegeben. Anschließend wurde für 30 Minuten bei 50°C gerührt, und dann auf Rückfluss (anfangs ca. 83°C) erhitzt.

Über einen Destillationsaufsatz mit kurzer Vigreux-Kolonne wurden anschließend niedrigsiedende Komponenten (Ethanol und Toluol) abdestilliert. Nach 4 Stunden wurden noch einmal 50 g (0,675 mol) 1-Butanol zugegeben und weiter niedrigsiedende Komponenten abdestilliert. Zuletzt wurde bei 140°C im Sumpf /113°C Kopf ein Gemisch aus 37% 1-Butanol und 63% Toluol (GC-Analyse) abgenommen.

Nach Abkühlen und Entfernen des sauren Katalysators verblieben 64,3 g eines Gemisches von 55,6% 1-Butyl methylphosphinat, 41,3% 1-Butanol und 3,1% Toluol (GC-Analyse gegen Standard), entsprechend einer Ausbeute von 35,75 g (0,263 mol) = 97,9% der Theorie.

Über eine fraktionierte Feindestillation im Vakuum wurde 1-Butyl methylphosphinat mit einer Reinheit von 98,5% erhalten.
Analytik: ³¹P-NMR (CDCl₃)
1-Butyl methylphosphinat 33,9 ppm
Diethyl methylphosphonit 177,8 ppm

### Beispiel 2: 1-Pentyl methylphosphinat (Methanphosphonigsäure-mono-n-pentylester)

20 g (0,145 mol) Diethyl methylphosphonit (Reinheit 99%) wurden mit 2,62 g (0,145 mol) Wasser und 64,1 g (0,727 mol) 1-Pentanol versetzt und in Gegenwart von 0,1 g an 96%iger Schwefelsäure ohne zusätzliches Lösungsmittel umgesetzt, wobei zu Beginn für 30 Minuten bei 50°C gerührt, und anschließend sukzessiv während 3 Stunden die Innentemperatur bis zuletzt auf Rückflusstemperatur von 1-Pentanol erhöht wurde. Währenddessen wurden niedrigsiedende Komponenten abdestilliert, zuletzt nur 1-Pentanol (GC-Analyse), das größtenteils abdestilliert wurde.

Gemäß GC-Analytik enthielt das resultierende Reaktionsgemisch kein Edukt mehr.

Aus dem Rohprodukt wurden nach Feindestillation im Vakuum 21,8 g (0,139 mol) an 1-Pentyl methylphosphinat (Reinheit 96%) erhalten, entsprechend einer Ausbeute von 95,8% der Theorie.
Analytik: ³¹P-NMR (CDCl₃): 1-Pentylmethylphosphinat 34,11 ppm

### Beispiel 3: 1-Butyl phenylphosphinat (Phenylphosphonigsäure-mono-n-butylester)

5,0 g (24,7 mmol) Diethyl phenylphosphonit (Reinheit 98%) unter Argon mit 9,72 g (131,17 mmol) 1-Butanol, 0,445 g (24,7 mmol) Wasser und 0,3 g Amberlyst® 15 (stark saurer Katalysator, Kunstharz-Ionenaustauscher) ohne zusätzliches Verdünnungsmittel bei 20°C versetzt und zum Rückfluss erhitzt. Anschließend wurden niedrigsiedende Komponenten über einen Destillationsaufsatz mit kurzer Vigreux-Kolonne abdestilliert bis zu einer Sumpftemperatur von 117-120°C. Danach wurden weitere 8,1 g (109,31 mmol) 1-Butanol zugesetzt und weiter niedrigsiedende Komponenten abdestilliert bei einer Sumpftemperatur von etwa 120°C. Die gesamte Umsetzung dauerte insgesamt etwa 5,5 Stunden. Der Verlauf wurde mittels GC-Analyse verfolgt. Am Ende verblieben 7,95 g Rohprodukt (laut ¹H-NMR 58,3%ig, der Rest war 1-Butanol). Das entsprach einer Ausbeute von 94,6% der Theorie.

Über eine Feindestillation im Vakuum konnte reines 1 -Butyl phenylphosphinat gewonnen werden.
Analytik: ³¹P-NMR (CDCl₃)
1-Butyl phenylphosphinat 25,1 ppm
Diethyl phenylphosphonit 151,2 ppm

### Beispiel 4: 2-Methylpropyl phenylphosphinat (Phenylphosphonigsäure-mono-2-methylpropylester)

Analog zu obigem Beispiel 2 wurde Diethyl phenylphosphonit (Reinheit 98%) mit Wasser (1 molares Äquivalent) und Isobutanol (8 molare Äquivalente) in Gegenwart einer katalytischen Mengen von konzentrierter Schwefelsäure (2 mol.-%, bezogen auf die eingesetzte Menge an Diethyl phenylphosphonit) umgesetzt.

Es wurde 2-Methylpropyl phenylphosphinat in einer Ausbeute von 95,0 % der Theorie erhalten, das nach Feindestillation eine Reinheit von 98% aufwies.
Analytik: ³¹P-NMR (CDCl₃): 2-Methylpropyl phenylphosphinat 25,4 ppm

### Beispiel 5: 2-Methylpropyl phenylphosphinat (Phenylphosphonigsäure-mono-2-methylpropylester)

Analog zu obigem Beispiel 3 wurde Diethyl phenylphosphonit (Reinheit 98%) mit Wasser (1 molares Äquivalent) und Isobutanol (20 molare Äquivalente, die in zwei etwa gleichen Portionen zugegeben wurden) in Gegenwart einer katalytischen Menge von Methansulfonsäure (1 Gew%, bezogen auf die eingesetzte Menge an Diethyl phenylphosphonit) umgesetzt.

Es wurde 2-Methylpropyl phenylphosphinat in einer Ausbeute von 97,0 % der Theorie erhalten, welches nach Feindestillation eine Reinheit von 98,5% aufwies.
Analytik: ³¹P-NMR (CDCl₃): 2-Methylpropyl phenylphosphinat 25,3 ppm

## Patentansprüche

1. Verfahren zur Herstellung einer Verbindung der Formel (I) 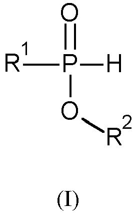 **dadurch gekennzeichnet, dass** eine Verbindung der Formel (II) 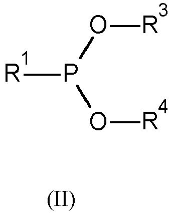 mit einer Verbindung der Formel (III)
R²-OH (III)
wobei jeweils gilt:
R¹ bedeutet (C₁-C₁₂)-Alkyl, (C₁-C₁₂)-Haloalkyl, (C₆-C₁₀)-Aryl, (C₆-C1₀)-Haloaryl, (C₇-C₁₀)-Aralkyl, (C₇-C₁₀)-Haloaralkyl, (C₄-C₁₀)-Cycloalkyl oder (C₄-C₁₀)-Halocycloalkyl,
R² bedeutet (C₃-C₁₂)-Alkyl, (C₃-C₁₂)-Haloalkyl, (C₆-C₁₀)-Aryl, (C₆-C1₀)-Haloaryl, (C₇-C₁₀)-Aralkyl, (C₇-C₁₀)-Haloaralkyl, (C₄-C₁₀)-Cycloalkyl oder (C₄-C₁₀)-Halocycloalkyl,
R³ und R⁴ bedeuten jeweils unabhängig voneinander Methyl oder Ethyl,
in Gegenwart eines sauren Katalysators und in Gegenwart von Wasser umgesetzt wird.

2. Verfahren nach Anspruch 1, **dadurch gekennzeichnet, dass** die insgesamt eingesetzte Menge an Wasser mindestens 0,8 molare Äquivalente beträgt, bevorzugt mindestens 0,9 molare Äquivalente, weiter bevorzugt mindestens 0,95 molare Äquivalente, jeweils bezogen auf die insgesamt eingesetzte Menge an Verbindungen der Formel (II).

3. Verfahren nach Anspruch 1 oder 2, **dadurch gekennzeichnet, dass** die insgesamt eingesetzte Menge an Wasser 1 bis 5 molare Äquivalente beträgt, bezogen auf die insgesamt eingesetzte Menge an Verbindungen der Formel (II).

4. Verfahren nach einem der Ansprüche 1 bis 3, **dadurch gekennzeichnet, dass** die insgesamt eingesetzte Menge an Wasser 1 bis 3 molare Äquivalente beträgt, bezogen auf die insgesamt eingesetzte Menge an Verbindungen der Formel (II).

5. Verfahren nach einem der Ansprüche 1 bis 4, **dadurch gekennzeichnet, dass** die insgesamt eingesetzte Menge an Wasser 1 bis 2 molare Äquivalente beträgt, bezogen auf die insgesamt eingesetzte Menge an Verbindungen der Formel (II).

6. Verfahren nach einem der Ansprüche 1 bis 5, **dadurch gekennzeichnet, dass** die insgesamt eingesetzte Menge an Verbindungen der Formel (III) 1 bis 25 molare Äquivalente beträgt, vorzugsweise 2 bis 20 molare Äquivalente, jeweils bezogen auf die insgesamt eingesetzte Menge an Verbindungen der Formel (II).

7. Verfahren nach einem der Ansprüche 1 bis 6, **dadurch gekennzeichnet, dass** die insgesamt eingesetzte Menge an Verbindungen der Formel (III) 3 bis 15 molare Äquivalente beträgt, vorzugsweise 4 bis 12 molare Äquivalente, bevorzugt 5 bis 10 molare Äquivalente, jeweils bezogen auf die insgesamt eingesetzte Menge an Verbindungen der Formel (II).

8. Verfahren nach einem der Ansprüche 1 bis 7, **dadurch gekennzeichnet, dass** der pKs-Wert des sauren Katalysators unter Standardbedingungen kleiner als 3 ist, vorzugsweise kleiner als 2 ist.

9. Verfahren nach einem der Ansprüche 1 bis 8, **dadurch gekennzeichnet, dass** einer oder der saure Katalysator ausgewählt ist aus der Gruppe bestehend aus H₃PO₃, H₂SO₄, HCl, HBr, HClO₄, Dichloressigsäure, Trichloressigsäure, Trifluoressigsäure, Methansulfonsäure, Trifluormethansulfonsäure, Benzolsulfonsäure, p-Toluolsulfonsäure, sauren Ionenaustauschern, sauren Polysiloxanen und sauren Zeolithen.

10. Verfahren nach einem der Ansprüche 1 bis 9, **dadurch gekennzeichnet, dass** die Umsetzung als Eintopfreaktion durchgeführt wird.

11. Verfahren nach einem der Ansprüche 1 bis 10, **dadurch gekennzeichnet, dass** die Umsetzung bei einer Temperatur im Bereich von 30 bis 140 °C erfolgt, bevorzugt bei einer Temperatur im Bereich von 40 bis 130 °C, und weiter bevorzugt bei einer Temperatur im Bereich von 50 bis 120 °C.

12. Verfahren nach einem der Ansprüche 1 bis 11, **dadurch gekennzeichnet, dass** die Umsetzung in einem inerten Verdünnungsmittel oder verdünnungsmittelfrei durchgeführt wird.

13. Verfahren nach einem der Ansprüche 1 bis 12, **dadurch gekennzeichnet, dass**
R¹ (C₁-C₆)-Alkyl, (C₁-C₆)-Haloalkyl, (C₆-C₈)-Aryl, (C₆-C₈)-Haloaryl, (C₇-C₁₀)-Aralkyl, (C₇-C₁₀)-Haloaralkyl, (C₅-C₈)-Cycloalkyl oder (C₅-C₈)-Halocycloalkyl bedeutet,
und
R² (C₃-C₈)-Alkyl, (C₃-C₈)-Haloalkyl, (C₆-C₈)-Aryl, (C₆-C₈)-Haloaryl, (C₇-C₁₀)-Aralkyl, (C₇-C₁₀)-Haloaralkyl, (C₅-C₈)-Cycloalkyl oder (C₅-C₈)-Halocycloalkyl bedeutet.

14. Verfahren nach einem der Ansprüche 1 bis 13, **dadurch gekennzeichnet, dass**
R¹ (C₁-C₄)-Alkyl, (C₁-C₄)-Haloalkyl oder (C₆-C₈)-Aryl, bedeutet, bevorzugt Methyl oder Ethyl,
R² (C₃-C₆)-Alkyl oder (C₃-C₆)-Haloalkyl bedeutet, bevorzugt (C₃-C₆)-Alkyl, dabei wiederum bevorzugt C₄-Alkyl oder C₅-Alkyl.

15. Verfahren zur Herstellung von biologisch aktiven Substanzen, die im pharmazeutischen bzw. agrochemischen Bereich eingesetzt werden können, bevorzugt zur Herstellung von phosphorhaltigen Aminosäuren, umfassend die folgenden Schritte (a) und (b):
(a) Herstellung einer Verbindung der Formel (I) wie in Anspruch 1, Anspruch 13 oder Anspruch 14 definiert gemäß einem in den Ansprüchen 1 bis 14 definierten Verfahren,
(b) Verwendung der in Schritt (a) hergestellten Verbindung der Formel (I) zur Herstellung von biologisch aktiven Substanzen, die im pharmazeutischen bzw. agrochemischen Bereich eingesetzt werden können, bevorzugt zur Herstellung von phosphorhaltigen Aminosäuren.

## Claims

1. A process for producing a compound of formula (I) 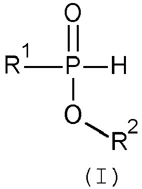 wherein a compound of formula (II) 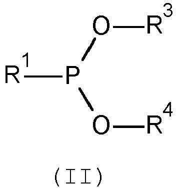 is reacted with a compound of formula (III)
R²-OH (III)
where in each case:
R¹ represents (C₁-C₁₂) -alkyl, (C₁-C₁₂) -haloalkyl, (C₆-C₁₀) -aryl, (C₆-C₁₀) -haloaryl, (C₇-C₁₀) -aralkyl, (C₇-C₁₀) -haloaralkyl, (C₄-C₁₀) -cycloalkyl or (C₄-C₁₀) - halocycloalkyl,
R² represents (C₃-C₁₂) -alkyl, (C₃-C₁₂) -haloalkyl, (C₆-C₁₀) -aryl, (C₆-C₁₀) -haloaryl, (C₇-C₁₀) -aralkyl, (C₇-C₁₀) -haloaralkyl, (C₄-C₁₀) -cycloalkyl or (C₄-C₁₀)-halocycloalkyl,
R³ and R⁴ each independently of one another represent methyl or ethyl,
in the presence of an acidic catalyst and in the presence of water.

2. The process according to claim 1, wherein the total amount of water used is at least 0.8 molar equivalents, preferably at least 0.9 molar equivalents, more preferably at least 0.95 molar equivalents, based in each case on the total amount of compounds of formula (II) used.

3. The process according to claim 1 or 2, wherein the total amount of water used is 1 to 5 molar equivalents, based on the total amount of compounds of formula (II) used.

4. The process according to any of claims 1 to 3, wherein the total amount of water used is 1 to 3 molar equivalents, based on the total amount of compounds of formula (II) used.

5. The process according to any of claims 1 to 4, wherein the total amount of water used is 1 to 2 molar equivalents, based on the total amount of compounds of formula (II) used.

6. The process according to any of claims 1 to 5, wherein the total amount of compounds of formula (III) used is 1 to 25 molar equivalents, preferably 2 to 20 molar equivalents, based in each case on the total amount of compounds of formula (II) used.

7. The process according to any of claims 1 to 6, wherein the total amount of compounds of formula (III) used is 3 to 15 molar equivalents, preferably 4 to 12 molar equivalents, preferably 5 to 10 molar equivalents, based in each case on the total amount of compounds of formula (II) used.

8. The process according to any of claims 1 to 7, wherein the pKa of the acidic catalyst under standard conditions is less than 3, preferably less than 2.

9. The process according to any of claims 1 to 8, wherein one or the acidic catalyst is selected from the group consisting of H₃PO₃, H₂SO₄, HC1, HBr, HClO₄, dichloroacetic acid, trichloroacetic acid, trifluoroacetic acid, methanesulphonic acid, trifluoromethanesulphonic acid, benzenesulphonic acid, p-toluenesulphonic acid, acidic ion exchangers, acidic polysiloxanes and acidic zeolites.

10. The process according to any of claims 1 to 9, wherein the reaction is carried out as a one-pot reaction.

11. The process according to any of claims 1 to 10, wherein the reaction is carried out at a temperature in the range from 30 to 140°C, preferably at a temperature in the range from 40 to 130°C and more preferably at a temperature in the range from 50 to 120°C.

12. The process according to any of claims 1 to 11, wherein the reaction is carried out in an inert diluent or diluent-free.

13. The process according to any of claims 1 to 12,
wherein
R¹ represents (C₁-C₆) -alkyl, (C₁-C₆) -haloalkyl, (C₆-C₈) -aryl, (C₆-C₈) -haloaryl, (C₇-C₁₀) -aralkyl, (C₇-C₁₀)-haloaralkyl, (C₅-C₈) -cycloalkyl or (C₅-C₈) - halocycloalkyl,
and
R² represents (C₃-C₈)-alkyl, (C₃-C₈)-haloalkyl, (C₆-C₈) -aryl, (C₆-C₈) -haloaryl, (C₇-C₁₀) -aralkyl, (C₇-C₁₀)-haloaralkyl, (C₅-C₈)-cycloalkyl or (C₅-C₈)-halocycloalkyl.

14. The process according to any of claims 1 to 13,
wherein
R¹ represents (C₁-C₄) -alkyl, (C₁-C₄) -haloalkyl or (C₆-C₈)-aryl, preferably methyl or ethyl,
R² represents (C₃-C₆)-alkyl or (C₃-C₆)-haloalkyl, preferably (C₃-C₆)-alkyl, preference among these in turn being given to C₄-alkyl or C₅-alkyl.

15. A process for producing biologically active substances which may be used in the pharmaceutical or agrochemical sector, preferably for producing phosphorus-containing amino acids, comprising the following steps (a) and (b):
(a) producing a compound of the formula (I) as defined in claim 1, claim 13 or claim 14 according to a process defined in claims 1 to 14,
(b) use of the compound of the formula (I) produced in step (a) for producing biologically active substances which may be used in the pharmaceutical or agrochemical sector, preferably for producing phosphorus-containing amino acids.

## Revendications

1. Procédé de fabrication d'un composé de formule (I) 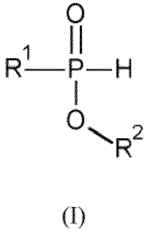 **caractérisé en ce qu'**un composé de formule (II) 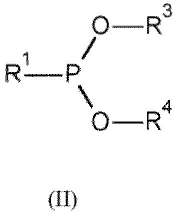 est mis en réaction avec un composé de formule (III) 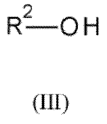 dans lesquelles
R¹ signifie alkyle en (C₁-C₁₂), haloalkyle en (C₁-C₁₂), aryle en (C₆-C₁₀), haloaryle en (C₆-C₁₀), aralkyle en (C₇-C₁₀), haloaralkyle en (C₇-C₁₀), cycloalkyle en (C₄-C₁₀) ou halocycloalkyle en (C₄-C₁₀),
R² signifie alkyle en (C₃-C₁₂), haloalkyle en (C₃-C₁₂), aryle en (C₆-C₁₀), haloaryle en (C₆-C₁₀), aralkyle en (C₇-C₁₀), haloaralkyle en (C₇-C₁₀), cycloalkyle en (C₄-C₁₀) ou halocycloalkyle en (C₄-C₁₀),
R³ et R⁴ signifient chacun indépendamment l'un de l'autre méthyle ou éthyle,
en présence d'un catalyseur acide et en présence d'eau.

2. Procédé selon la revendication 1, **caractérisé en ce que** la quantité totale utilisée d'eau est d'au moins 0,8 équivalent molaire, de préférence d'au moins 0,9 équivalent molaire, de manière davantage préférée d'au moins 0,95 équivalent molaire, à chaque fois par rapport à la quantité totale utilisée de composés de formule (II).

3. Procédé selon la revendication 1 ou 2, **caractérisé en ce que** la quantité totale utilisée d'eau est de 1 à 5 équivalents molaires, par rapport à la quantité totale utilisée de composés de formule (II).

4. Procédé selon l'une quelconque des revendications 1 à 3, **caractérisé en ce que** la quantité totale utilisée d'eau est de 1 à 3 équivalents molaires, par rapport à la quantité totale utilisée de composés de formule (II).

5. Procédé selon l'une quelconque des revendications 1 à 4, **caractérisé en ce que** la quantité totale utilisée d'eau est de 1 à 2 équivalents molaires, par rapport à la quantité totale utilisée de composés de formule (II).

6. Procédé selon l'une quelconque des revendications 1 à 5, **caractérisé en ce que** la quantité totale utilisée de composés de formule (III) est de 1 à 25 équivalents molaires, de préférence de 2 à 20 équivalents molaires, à chaque fois par rapport à la quantité totale utilisée de composés de formule (II).

7. Procédé selon l'une quelconque des revendications 1 à 6, **caractérisé en ce que** la quantité totale utilisée de composés de formule (III) est de 3 à 15 équivalents molaires, de préférence de 4 à 12 équivalents molaires, de préférence de 5 à 10 équivalents molaires, à chaque fois par rapport à la quantité totale utilisée de composés de formule (II).

8. Procédé selon l'une quelconque des revendications 1 à 7, **caractérisé en ce que** la valeur de pKs du catalyseur acide en conditions standard est inférieure à 3, de préférence inférieure à 2.

9. Procédé selon l'une quelconque des revendications 1 à 8, **caractérisé en ce qu'**un ou le catalyseur acide est choisi dans le groupe constitué par H₃PO₃, H₂SO₄, HCl, HBr, HClO₄, l'acide dichloroacétique, l'acide trichloroacétique, l'acide trifluoroacétique, l'acide méthane-sulfonique, l'acide trifluorométhane-sulfonique, l'acide benzène-sulfonique, l'acide p-toluène-sulfonique, les échangeurs d'ions acides, les polysiloxanes acides et les zéolithes acides.

10. Procédé selon l'une quelconque des revendications 1 à 9, **caractérisé en ce que** la réaction est réalisée sous la forme d'une réaction monotope.

11. Procédé selon l'une quelconque des revendications 1 à 10, **caractérisé en ce que** la réaction a lieu à une température dans la plage allant de 30 à 140 °C, de préférence à une température dans la plage allant de 40 à 130 °C, et de manière davantage préférée à une température dans la plage allant de 50 à 120 °C.

12. Procédé selon l'une quelconque des revendications 1 à 11, **caractérisé en ce que** la réaction est réalisée dans un diluant inerte ou sans diluant.

13. Procédé selon l'une quelconque des revendications 1 à 12, **caractérisé en ce que**
R¹ signifie alkyle en (C₁-C₆), haloalkyle en (C₁-C₆), aryle en (C₆-C₈), haloaryle en (C₆-C₈), aralkyle en (C₇-C₁₀), haloaralkyle en (C₇-C₁₀), cycloalkyle en (C₅-C₈) ou halocycloalkyle en (C₅-C₈),
et
R² signifie alkyle en (C₃-C₈), haloalkyle en (C₃-C₈), aryle en (C₆-C₈), haloaryle en (C₆-C₈), aralkyle en (C₇-C₁₀), haloaralkyle en (C₇-C₁₀), cycloalkyle en (C₅-C₈) ou halocycloalkyle en (C₅-C₈).

14. Procédé selon l'une quelconque des revendications 1 à 13, **caractérisé en ce que**
R¹ signifie alkyle en (C₁-C₄), haloalkyle en (C₁-C₄) ou aryle en (C₆-C₈), de préférence méthyle ou éthyle,
R² signifie alkyle en (C₃-C₆) ou haloalkyle en (C₃-C₆), de préférence alkyle en (C₃-C₆), de manière encore préférée alkyle en C₄ ou alkyle en C₅.

15. Procédé de fabrication de substances biologiquement actives, qui peuvent être utilisées dans le domaine pharmaceutique ou agrochimique, de préférence pour la fabrication d'acides aminés contenant du phosphore, comprenant les étapes (a) et (b) suivantes :
(a) la fabrication d'un composé de formule (I) tel que défini dans la revendication 1, la revendication 13 ou la revendication 14 par un procédé défini dans les revendications 1 à 14,
(b) l'utilisation du composé de formule (I) fabriqué à l'étape (a) pour la fabrication de substances biologiquement actives, qui peuvent être utilisées dans le domaine pharmaceutique ou agrochimique, de préférence pour la fabrication d'acides aminés contenant du phosphore.
